# EUROPÄISCHE PATENTSCHRIFT

(11) **EP 2 413 856 B1**
(45) Veröffentlichungstag und Bekanntmachung des Hinweises auf die Patenterteilung: **06.11.2019**
(21) Anmeldenummer: 09776498.9
(22) Anmeldetag: 01.04.2009
(51) Int. Cl.: A61F 9/008, A61F 9/009

(54) **VORRICHTUNG FÜR DIE BEHANDLUNG EINES AUGES MIT LASERSTRAHLUNG**
DEVICE FOR THE LASER RADIATION TREATMENT OF AN EYE
DISPOSITIF DESTINÉ AU TRAITEMENT DE L'OEIL PAR FAISCEAU LASER

(43) Veröffentlichungstag der Anmeldung: 08.02.2012
(73) Patentinhaber: WaveLight GmbH, 91058 Erlangen (DE)
(72) Erfinder: DONITZKY, Christof, 90542 Eckental (DE); KITTELMANN, Olaf, 14109 Berlin (DE)
(74) Vertreter: von Hellfeld, Axel
(86) Internationale Anmeldenummer: PCT/EP2009/002383
(87) Internationale Veröffentlichungsnummer: WO 2010/112041

(56) Entgegenhaltungen:
- EP-A- 1 844 745
- WO-A-2007/022993
- WO-A-2008/098381
- WO-A-2009/033111
- WO-A1-2008/150331
- US-A1- 2009 069 794

## Beschreibung

Die Erfindung betrifft eine Vorrichtung für die Behandlung eines Auges mit Laserstrahlung.

In der refraktiven ophthalmologischen Chirurgie werden durch Eingriffe am Auge eines Patienten die Brechungs- und Abbildungseigenschaften des Auges zur Korrektur oder Linderung von Sehfehlern verändert. Bekannt ist insbesondere das LASIK-Verfahren, bei dem die Hornhaut (Kornea) des Auges neu geformt wird. Beim herkömmlichen LASIK-Verfahren wird in einem ersten Schritt mit einem mechanischen Mikrokeratom ein flacher Hornhautschnitt gesetzt, um so einen sogenannten "Flap" (Deckelchen) zu erzeugen, der auf einer Seite fest mit der Hornhaut verbunden bleibt, so dass er aufgeklappt werden kann, um darunter liegendes Hornhautgewebe (Stroma) freizulegen. In dem freigelegten Stroma wird dann die sogenannte Ablation, also die Entfernung von Gewebe mittels üblicherweise Excimerlaserstrahlung durchgeführt, woraufhin dann der Flap zurückgeklappt wird und wieder verheilt. Dabei bleibt das Epithel weitgehend unverletzt und der Heilungsprozess erfolgt relativ kurz und schmerzfrei. Bei einem herkömmlichen mechanischen Mikrokeratom oszilliert eine scharfe Klinge.

In jüngerer Zeit wird zum Schneiden des Flaps das mechanische Mikrokeratom zunehmend durch Laserstrahlung ersetzt. Die Laserstrahlung wird unterhalb der Oberfläche der Hornhaut fokussiert und auf einer Bahn geführt, wobei die Leistungsdichten so hoch sind, dass durch sogenannte photodisruptive Effekte ein kontinuierlicher Schnitt entsteht. Um die hohen Leistungsdichten zu erhalten, werden extrem kurze Laserpulse im Femtosekundenbereich eingesetzt, weshalb dieses Verfahren auch als Fs-LASIK bezeichnet wird. Die vorliegende Erfindung betrifft insbesondere diese Fs-LASIK, darüber hinaus aber auch jedes andere Verfahren zur Behandlung eines Auges mit Laserstrahlung, bei dem auch die Strahlung in Raum und Zeit in Bezug auf das Auge gemäß einem sogenannten Bearbeitungsprogramm geführt wird. Es versteht sich, dass diese Steuerung der Laserstrahlung in Raum und Zeit in Bezug auf einen genau definierten und reproduzierbaren Bezugspunkt des Auges erfolgen muss. Als einen solchen Bezugspunkt wählt man in aller Regel ein sogenanntes Zentrum des Auges, also einen zentral gelegenen Punkt, der als Referenz dient für die örtliche Führung der Laserstrahlung über das Auge. Da die Laserstrahlung bei den hier in Rede stehenden Verfahren in aller Regel auf kleine "Spots" fokussiert wird, werden also die Orte eines jeden Spots in Bezug auf das genannte Zentrum als Referenzpunkt ausgerichtet. Die vorliegende Erfindung betrifft insbesondere die Fs-LASIK, aber auch andere Augenbehandlungen, bei denen Laserstrahlung in Bezug auf das Auge in genau definierter Weise zu positionieren ist, wie etwa bei der Keratoplastik (z. B. anteriore oder posteriore lamellare Keratoplastik, die perforierende Keratoplastik bei Hornhauttransplantationen), die Fs-Lentikel-Extraktion zur Refraktionskorrektur, das Schneiden von interkornealen Ringsegmenten zur Stabilisierung von Keratokonus und Hornhautvorwölbung, Kataraktinzisionen, Presbyopie-Schnitt in der Augenlinse, intrastromale Inlays, die Keratomie bei Astigmatismus, die korneale Resektion etc.

Im Stand der Technik erfolgt die Zentrierung des chirurgischen Behandlungsortes üblicherweise durch Justierung des sogenannten Applikators, wie einem Saugring, der auf einer Seite durch Ansaugen mit dem Auge verbunden wird und auf der anderen Seite eine Aufnahme aufweist, an welche eine Fokussieroptik ankoppelbar ist, mit welcher die Laserstrahlung auf oder in die Kornea fokussiert wird. Dabei nimmt der Chirurg die Positionierung des Applikators (Saugrings) auf dem Auge nach "Augenmaß" vor, ggf. unter Nutzung optischer Vergrößerungseinrichtungen. Der Chirurg versucht, den Applikator möglichst zentrisch in Bezug auf bestimmte Konturen des Auges zu platzieren. Anhalt für diese Zentrierung nach Augenmaß kann zum Beispiel die Pupille oder die Iris sein. Allerdings hängt bei diesem Stand der Technik die optimale Positionierung und Zentrierung des Applikators und damit des chirurgischen Behandlungsortes relativ zum Auge stark von den subjektiven Fähigkeiten des Chirurgen ab. Mit anderen Worten: Bei dieser herkömmlichen Positionierung des Applikators können suboptimale Verhältnisse auftreten. Der Stand der Technik findet sich beispielsweise in US 2009/069794 und WO 2008/150331.

Der Erfindung liegt die Aufgabe zugrunde, eine Vorrichtung für die Behandlung eines Auges mit Laserstrahlung bereitzustellen, bei dem die Strahlung gemäß einem Bearbeitungsprogramm in Raum und Zeit in Bezug auf das Auge derart gesteuert wird, dass für die Steuerung der Laserstrahlung ein genauer und zuverlässig reproduzierbarer Bezugspunkt zur Verfügung steht. Die Erfindung wird durch die Ansprüche definiert.

Eine erfindungsgemäße Vorrichtung zur Lösung dieser Aufgabe weist folgendes auf:
Eine Laserstrahlungsquelle zur Erzeugung von Laserstrahlung, Mittel zum Richten der Laserstrahlung auf das Auge zwecks eines ophthalmologischen Eingriffs am oder im Auge, eine Steuerung zum Steuern der Laserstrahlung in Raum und Zeit in Bezug auf das Auge gemäß einem Bearbeitungsprogramm, das auf ein Zentrum des Auges ausgerichtet ist, eine Kamera, die ein Merkmal des Auges aufnimmt, und eine Bildverarbeitungseinheit, die aus der Kameraufnahme eine Information über das Zentrum des Auges ableitet und diese Information in die Steuerung eingibt, wodurch die Steuerung die Laserstrahlung gemäß dem Bearbeitungsprogramm und in Abhängigkeit von dem abgeleiteten Zentrum des Auges steuert.

Die Erfindung ermöglicht damit eine exakte Zentrierung der chirurgischen Behandlung in Bezug auf das Zielgewebe (Kornea). Hierzu wird ein Kamerasystem eingesetzt, welches anhand eines Augenmerkmals, d.h. einer vorgegebenen anatomischen Struktur des Auges, den Behandlungsort mittels einer Bildverarbeitung automatisch erkennt, d.h. ohne Einfluss subjektiver, auf den jeweiligen Chirurgen zurückgehender Einflüsse. Geeignete Merkmale für eine Bildverarbeitung zur Ermittlung eines Referenzpunktes, insbesondere eines Zentrums für die Bearbeitung, sind geometrische Strukturen des Auges, aus denen mit einer Bildverarbeitung automatisch ein Zentrum abgeleitet (ermittelt) werden kann, wie zum Beispiel die Pupille, deren Mitte als Zentrum definiert werden kann, die Iris-Struktur oder auch die Limbusstruktur. Auch kann alternativ oder zusätzlich im hinteren Augenabschnitt die Struktur der Retina erfasst werden und es können aus der Anordnung von Blutgefäßen im retinalen Bereich und/oder der Orientierung der Fovea zur Papille Aussagen über einen Referenzpunkt für die Laserbearbeitung abgeleitet werden.

Eine besondere Ausgestaltung der Erfindung nimmt Bezug auf den oben eingeführten Applikator, also zum Beispiel einen Saugring. Solche Saugring-Techniken sind zum Beispiel in der US 5,549,632 WO 03/002008 A1, und PCT/EP2008/006962 beschrieben. Wird die Erfindung zusammen mit einem Applikator eingesetzt, dann ist die Kamera eingerichtet, den Applikator und zumindest ein geometrisch-strukturelles Merkmal des Auges aufzunehmen, woraufhin die Bildverarbeitungseinheit aus dieser sowohl den Applikator als auch die Augenstruktur betreffenden Aufnahme eine örtliche Beziehung zwischen der Lage des Applikators in Bezug auf das Auge ableitend und an die Lasersteuerung ein entsprechendes Signal abgibt, woraufhin dann die Steuerung die Laserstrahlung in Abhängigkeit von diesem Signal in Bezug auf das Auge steuert. Das bedeutet, dass eine eventuelle suboptimale Positionierung des Applikators in Bezug auf das Auge rechnerisch bei der Steuerung der Laserstrahlung kompensiert wird, also zum Beispiel dann, wenn das Steuerprogramm für die Steuerung der Laserstrahlung gemäß einem bestimmten Bearbeitungsprogramm zunächst auf das Zentrum des Applikators ausgerichtet ist, aber durch die Bildverarbeitung festgestellt wird, dass der Applikator nicht optimal zentrisch in Bezug auf das Auge positioniert ist, anschließend das Bearbeitungsprogramm nicht mehr auf das Zentrum des Applikators ausgerichtet wird, sondern auf das durch die Bildverarbeitung tatsächlich ermittelte Augenzentrum.

Alternativ kann erfindungsgemäß die Kamera mit dem Bildverarbeitungsprogramm auch so eingesetzt werden, dass dann, wenn die Mitte des Applikators nicht in idealer Weise mit dem gewünschten Referenzpunkt für die Laserbehandlung (also zum Beispiel dem Zentrum der Pupille) zusammenfällt, dem Chirurgen eine Hilfe gegeben wird dahingehend, wie der Applikator am besten neu auf dem Auge so positioniert wird, dass der Referenzpunkt auf der Mittelachse des Applikators zu liegen kommt. Dann kann der Chirurg den Applikator zunächst lösen und sodann neu entsprechend dieser Angabe positionieren. Bei einem vollmechanisierten System kann dieses Lösen und Neuansetzen des Applikators auch voll mechanisch geschehen.

Die Erfindung eignet sich besonders zum Einsatz bei der oben erläuterten Fs-LASIK zum Schneiden des Flaps derart, dass die Geometrie und Positionierung des Flap-Schnittes genau auf einen Referenzpunkt am Auge so ausgerichtet wird, dass nach Hochklappen des Flaps ein möglichst großer und optimal gelegener Bereich des Stroma zur Verfügung steht, um die gewünschte Ablation durchzuführen.

Nachfolgend werden Ausführungsbeispiele der Erfindung anhang der Zeichnung näher erläutert. Es zeigt
Fig. 1 schematisch ein Ausführungsbeispiel einer Vorrichtung für die Behandlung eines Auges mit Laserstrahlung;
Fig. 2 schematisch die Draufsicht auf ein Auge und die Positionierung eines Flap-Schnittes für Fs-LASIK;
Fig. 3 eine schematische Ansicht entsprechend Fig. 2, wobei der Flap-Schnitt optimiert positioniert ist; und
Fig. 4 eine andere Konstellation mit optimiertem Flap-Schnitt.

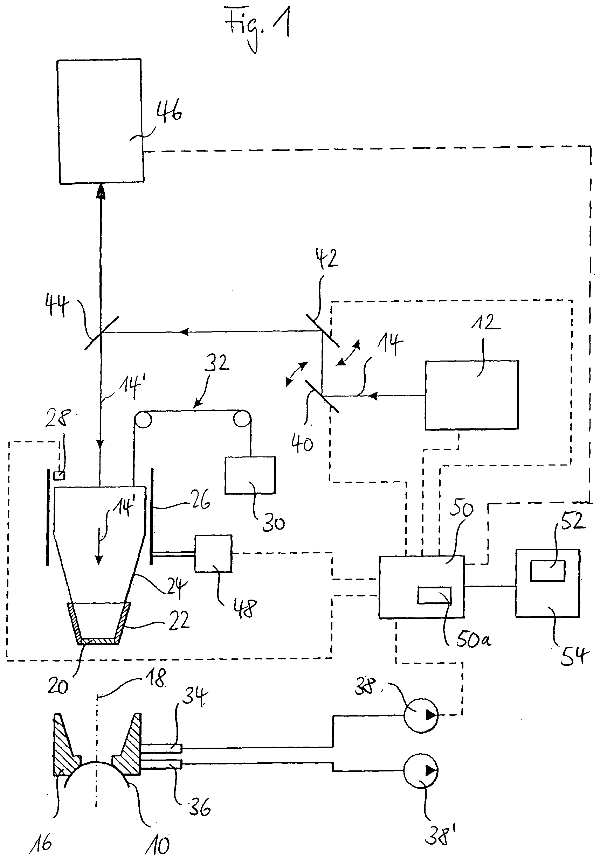

In Fig. 1 ist das mit Laserstrahlung zu behandelnde Auge mit dem Bezugszeichen 10 schematisch dargestellt. Bei diesem Ausführungsbeispiel dient ein Laser 12 der Erzeugung von Femtosekundenpulsen. Die Laserstrahlung 14 wird über weiter unten näher beschriebene Mittel auf das Auge 10 gerichtet.

Mit einem als solches bekannten Saugring 16 wird das Auge fixiert und auf der Zentralachse 18 des Saugringes 16 wird eine Aplanationslinse 20 in eine Aufnahme des Saugringes eingeführt, d.h. aus der in Figur 1 gezeigten Position nach unten abgesenkt. Dabei koppelt eine Schnittstelleneinheit 22 eine Fokussieroptik 24 an den Saugring 16. Die Fokussieroptik 24 wird in einer Halterung 26 geführt. Die Führung erfolgt mittels eines Ortssensors 28, wobei die Fokussieroptik 24 über ein Gegengewicht 30 und eine Seil-/Rollenanordnung bzw. ein Drehgelenk frei schwebend aufgehängt ist, um eine das Auge so gut wie nicht belastende Ankopplung der Schnittstelleneinheit mit der Fokussieroptik 24 an das Auge 10 zu ermöglichen.

Der Saugring 16 wird mittels als solches bekannter Anschlussstutzen 34, 36 und Vakuumpumpen 38 fixiert.

Die von dem Laser 12 erzeugte Laserstrahlung 14 wird über als solches bekannte Spiegel 40, 42, 44 in die Fokussieroptik 24 gerichtet. Eine Computersteuerung 50 steuert sämtliche steuerbaren Komponenten des Systems, wobei die Steuerverbindungen in Fig. 1 durch gestrichelte Linien angedeutet sind. In einem Speicher 54 ist ein Steuerprogramm abgelegt, d.h. ein Bearbeitungsprogramm für die Steuerung der Laserstrahlung 14' in Raum und Zeit in Bezug auf das Auge 10.

Eine Kamera 46 ist oberhalb eines für vom Auge 10 kommende Strahlung durchlässigen Spiegels 44 angeordnet, so dass mit der Kamera 46 geometrische Strukturen am Auge 10 digital aufgenommen werden können, z.B. eine CCD-/CMOS-Kamera. In der Computersteuerung 50 befindet sich eine Bildverarbeitungseinheit 50a, welche von der Kamera 46 gelieferte Bilder verarbeitet, um aus einer vorgegebenen geometrischen Struktur des Auges, wie z.B. der Pupille, einen Referenzpunkt, insbesondere ein Zentrum, abzuleiten gemäß dem die Steuerung 50 das Bearbeitungsprogramm 52 abarbeitet. Die Anordnung gemäß Fig. 1 ist in der internationalen Patentanmeldung PCT/EP2008/006962 näher erläutert, die hier durch Bezugnahme voll eingeschlossen ist.

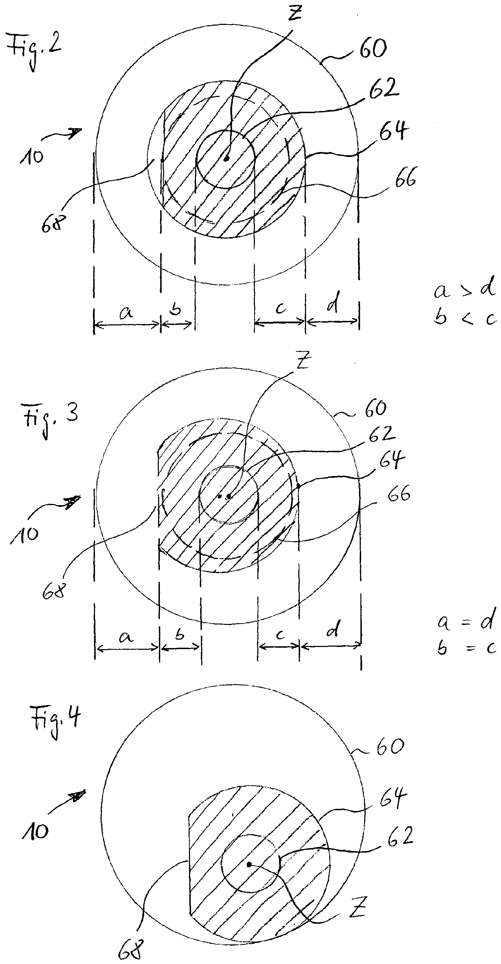

Die Figuren 2, 3 und 4 zeigen in Draufsicht auf ein Auge 10 schematisch Einzelheiten der Bildverarbeitung mit der Kamera 46 und der Bildverarbeitungseinheit 50a.

Die Figuren 2, 3 und 4 zeigen schematisch den Umfang 60 der optisch nutzbaren Fläche der Kornea. Die Pupille ist mit dem Bezugszeichen 62 markiert. 64 bezeichnet den Umfang eines möglichen Flap-Schnittes, also den in den Figuren schraffierten Bereich. Das Bezugszeichen 66 markiert in gestrichelter Linie den Ablationsbereich, also denjenigen Bereich der Kornea, in dem nach Zurückklappen des Deckelchens (gemäß dem Umfang 64) im Stroma Korneagewebe ablatiert werden soll. Wie eingangs beschrieben, hat das Deckelchen einen Verbindungsbereich mit der Kornea, der nicht geschnitten wird, üblicherweise als Hinge (Scharnier) bezeichnet, was in den Figuren mit dem Bezugszeichen 68 markiert ist.

Gemäß Fig. 2 hat die Pupille 62 ein Zentrum Z. Der Scharnierbereich 68 für das Deckelchen ist für die Laserablation nicht nutzbar, so dass bei konzentrischer Anordnung des Flap-Umfangs 64 in Bezug auf die Pupille 62 ein suboptimaler Bereich für die Ablation entsteht. Durch das Scharnier 68 ist die Strecke a gemäß Fig. 2 größer als die Strecke d auf der dem Scharnier 68 gegenüberliegenden Seite der Kornea. Auch ist der Abstand b zwischen dem Scharnier 68 und der Pupille 62 auf der Scharnierseite kleiner als der entsprechende Abstand c auf der gegenüberliegenden Seite, wie bei Fig. 2 angegeben ist.

Die Kamera 46 nimmt beim dargestellten Ausführungsbeispiel die Pupille 62 auf und leitet aus diesem Merkmal die Position des Zentrums Z der Pupille ab gemäß einem als solches bekannten Algorithmus, etwa analog einer sogenannten Schwerpunktbildung bei nicht vollständig kreisförmiger Pupillenform. Die Bildverarbeitungseinheit 50a im Steuerungsrechner 50 verarbeitet nun die Bildaufnahme so, dass eine maximale Ablationszone 66 dadurch erreicht wird, dass gemäß Fig. 3 der dort definierte Abstand a gleich dem Abstand d wird und analog der Abstand b gleich dem Abstand c, wobei diese Abstände, wie zeichnerisch dargestellt, immer senkrecht zur Kante des Scharniers 68 gemessen werden. Somit entsteht ein Flap-Umfang 64, der nicht genau zentrisch ist in Bezug auf das Pupillenzentrum Z. Es wird also zur Gewinnung einer maximalen Ablationszone der Umfang 64 für den Flap-Schnitt in Bezug auf das Pupillenzentrum Z versetzt, und zwar automatisch mit Hilfe der Kamera 46 und der Bildverarbeitungseinheit 50a wird das Bearbeitungsprogramm zur Steuerung der Laserstrahlung 14 zur Erzeugung des Flap-Schnittes so geometrisch in Bezug auf den Referenzpunkt Z verschoben, dass die in Fig. 3 eingezeichneten Abstände a, b, c, d die angegebenen Gleichheitsrelationen zumindest annähernd erfüllen.

Fig. 4 zeigt schematisch eine etwas extreme Situation bei einem gegebenen Patientenauge mit in Bezug auf den Mittelpunkt der optisch nutzbaren Fläche 60 stark verschobener Pupille 62. Auch bei diesem Ausführungsbeispiel wird der Flap-Umfang 64 in Bezug auf das Pupillenzentrum Z nicht konzentrisch gewählt, sondern das Zentrum des Flap-Umfangs 64 wird in Bezug auf das Zentrum Z der Pupille in einer Richtung senkrecht zur Kante des Scharniers 68 verschoben, wobei eine Einschränkung bezüglich des maximal möglichen flap-Durchmessers dadurch gegeben ist, dass der flap-Durchmesser nur innerhalb der optisch nutzbaren Fläche 60 erzeugt werden kann.

Die Ausführungsbeispiele zeigen eine objektive, d.h. automatisierte Positionierung des Ablationsbereichs 66.

## Patentansprüche

1. Vorrichtung für die Behandlung eines Auges mit Laserstrahlung, folgendes aufweisend:
a) eine Laserstrahlungsquelle (12), die eingerichtet ist, um Laserstrahlung (14, 14') zu erzeugen,
b) Mittel (20, 24, 40, 42, 44), die eingerichtet sind, um die Laserstrahlung (14, 14') auf das Auge (10) zwecks eines ophthalmologischen Eingriffs am oder im Auge zu richten,
b1) - einen Applikator (16), der mit dem Auge (10) in Eingriff bringbar ist, um die genannten Mittel zum Richten der Laserstrahlung auf das Auge auszurichten,
c) eine Steuerung (50), die eingerichtet ist, um die Laserstrahlung (14, 14') in Raum und Zeit in Bezug auf das Auge (10) gemäß einem Bearbeitungsprogramm (52) zu steuern,
d) eine Kamera (46), die eingerichtet ist, um den Applikator und zumindest ein Merkmal (62) des Auges (10) aufzunehmen,
e) eine Bildverarbeitungseinheit (50a), die eingerichtet ist, um aus der Kameraaufnahme eine Information über das Auge (10) ableitet und diese Information in die Steuerung einzugeben, wodurch
f) die Steuerung (50) eingerichtet ist, um die Laserstrahlung gemäß dem Bearbeitungsprogramm und in Abhängigkeit von der in Schritt (e) abgeleiteten Information zu steuern,
g) wobei die Bildverarbeitungseinheit (50a) eingerichtet ist, um:
- aus der Kameraaufnahme eine Information über das Zentrum (Z) des Auges abzuleiten und
- aus der Kameraaufnahme eine örtliche Beziehung zwischen dem Applikator (16) und dem Merkmal (62) des Auges abzuleiten und ein dieser Beziehung entsprechendes Signal an die Steuerung (50) abzugeben, und
h) wobei die Steuerung (50) eingerichtet ist, um die Laserstrahlung (14, 14') gemäß dem Bearbeitungsprogramm und in Abhängigkeit von der in Schritt (g) abgeleiteten Information über das Zentrum (Z) des Auges und in Abhängigkeit von dem der Beziehung entsprechenden Signal zu steuern.

2. Vorrichtung nach Anspruch 1, wobei die Steuerung (50) eingerichtet ist, um bei der Steuerung der Laserstrahlung (14) den Versatz des Mittelpunktes des Applikators (16) vom Mittelpunkt (Z) des Auges zu kompensieren.

3. Vorrichtung nach Anspruch 1, wobei das Merkmal des Auges dessen Pupille (52) betrifft.

4. Vorrichtung nach Anspruch 1, wobei das Merkmal des Auges dessen Iris betrifft.

5. Vorrichtung nach Anspruch 1, wobei das Merkmal des Auges dessen Retina betrifft.

6. Vorrichtung nach Anspruch 1, wobei das Merkmal des Auges dessen Kornea und/oder Pupille betrifft.

7. Vorrichtung nach Anspruch 1, wobei der Applikator (16) ein Saugring ist.

8. Vorrichtung nach Anspruch 1, wobei der ophthalmologische Eingriff das Schneiden eines Deckelchens, "flap", in der Kornea es Auges betrifft.

9. Vorrichtung nach Anspruch 1, wobei die Laserstrahlungsquelle (12) eingerichtet ist, um Femtosekundenpulse zu erzeugen.

10. Vorrichtung nach Anspruch 8, wobei der Umfang (64) des Deckelchens, "flap", in Bezug auf das Zentrum der Pupille des Auges in Richtung senkrecht zur Kante eines Scharniers (68) verschoben ist, mit dem das Deckelchen an der Kornea des Auges aufklappbar befestigt ist.

## Claims

1. Apparatus for treating an eye with laser radiation, comprising:
a) a laser radiation source (12) which is configured to produce laser radiation (14, 14'),
b) means (20, 24, 40, 42, 44) which are configured to direct the laser radiation (14, 14') onto the eye (10) for the purposes of an ophthalmological intervention on or in the eye,
b1) an applicator (16) that can be made to engage with the eye (10) for the purposes of alignment of the aforementioned means for directing the laser radiation onto the eye,
c) a controller (50) which is configured to control the laser radiation (14, 14') in space and time in relation to the eye (10) according to a treatment program (52),
d) a camera (46) which is configured to record the applicator and at least one feature (62) of the eye (10),
e) an image processing unit (50a) which is configured to derive information about the eye (10) from the camera recording and enter this information into the controller, as result of which
f) the controller (50) is configured to control the laser radiation according to the treatment program and depending on the information derived in step (e),
g) wherein the image processing unit (50a) is configured:
- to derive information about the centre (Z) of the eye from the camera recording and
- to derive a spatial relationship between the applicator (16) and the feature (62) of the eye from the camera recording and to transmit a signal corresponding to this relationship to the controller (50), and
h) wherein the controller (50) is configured to control the laser radiation (14, 14') according to the treatment program and depending on the information about the centre (Z) of the eye derived in step (g) and depending on the signal corresponding to the relationship.

2. Apparatus according to Claim 1, wherein the controller (50) is configured to compensate the offset of the centre of the applicator (16) from the centre (Z) of the eye when controlling the laser radiation (14).

3. Apparatus according to Claim 1, wherein the feature of the eye relates to the pupil (52) thereof.

4. Apparatus according to Claim 1, wherein the feature of the eye relates to the iris thereof.

5. Apparatus according to Claim 1, wherein the feature of the eye relates to the retina thereof.

6. Apparatus according to Claim 1, wherein the feature of the eye relates to the cornea and/or pupil thereof.

7. Apparatus according to Claim 1, wherein the applicator (16) is a suction ring.

8. Apparatus according to Claim 1, wherein the ophthalmological intervention relates to cutting a flap into the cornea of the eye.

9. Apparatus according to Claim 1, wherein the laser radiation source (12) is configured to produce femtosecond laser pulses.

10. Apparatus according to Claim 8, wherein the circumference (64) of the flap is displaced relative to the centre of the pupil of the eye in the direction perpendicular to the edge of a hinge (68), by means of which the flap is fastened, so as to be able to be folded open, to the cornea of the eye.

## Revendications

1. Dispositif de traitement d'un œil avec un rayonnement laser, ledit dispositif comprenant :
a) une source de rayonnement laser (12) adaptée pour générer un rayonnement laser (14, 14'),
b) des moyens (20, 24, 40, 42, 44) adaptés pour diriger le rayonnement laser (14, 14') sur l'œil (10) en vue d'une intervention ophtalmologique au niveau de l'œil ou dans l'œil,
b1) - un applicateur (16) qui peut être amené en engagement avec l'œil (10) pour orienter lesdits moyens de manière à diriger le rayonnement laser vers l'œil,
c) une commande (50) adaptée pour commander le rayonnement laser (14, 14') dans l'espace et dans le temps par rapport à l'œil (10) selon un programme de traitement (52),
d) une caméra (46) adaptée pour enregistrer l'applicateur et au moins un élément caractéristique (62) de l'œil (10),
e) une unité de traitement d'image (50a) qui est adaptée pour obtenir de l'enregistrement par caméra une information concernant l'œil (10) et pour entrer cette information dans la commande,
f) la commande (50) étant ainsi adaptée pour commander le rayonnement laser en fonction du programme de traitement et en fonction de l'information obtenue à l'étape (e),
g) l'unité de traitement d'image (50a) étant adaptée pour :
- obtenir de l'enregistrement par caméra une information sur le centre (Z) de l'œil et
- obtenir de l'enregistrement par caméra une relation locale entre l'applicateur (16) et l'élément caractéristique (62) de l'œil et délivrer à la commande (50) un signal correspondant à cette relation, et
h) la commande (50) étant adaptée pour commander le rayonnement laser (14, 14') conformément au programme de traitement et en fonction de l'information, obtenue à l'étape (g), relative au centre (Z) de l'œil et en fonction du signal correspondant à la relation.

2. Dispositif selon la revendication 1, la commande (50) étant adaptée pour compenser le déplacement du point central de l'applicateur (16) par rapport au point central (Z) de l'œil lors de la commande du rayonnement laser (14).

3. Dispositif selon la revendication 1, l'élément caractéristique de l'œil concernant sa pupille (52).

4. Dispositif selon la revendication 1, l'élément caractéristique de l'œil concernant son iris.

5. Dispositif selon la revendication 1, l'élément caractéristique de l'œil concernant sa rétine.

6. Dispositif selon la revendication 1, l'élément caractéristique de l'œil concernant sa cornée et/ou sa pupille.

7. Dispositif selon la revendication 1, l'applicateur (16) étant une bague d'aspiration.

8. Dispositif selon la revendication 1, l'intervention ophtalmologique concernant la découpe d'un opercule, « rabat », dans la cornée de l'œil.

9. Dispositif selon la revendication 1, la source de rayonnement laser (12) étant adaptée pour générer des impulsions de l'ordre de la femto-seconde.

10. Dispositif selon la revendication 8, la périphérie (64) de l'opercule, « rabat », étant déplacée par rapport au centre de la pupille de l'œil dans une direction perpendiculaire au bord d'une charnière (68) avec laquelle l'opercule est fixé à la cornée de l'œil de manière relevable.
